(19) Europäisches Patentamt
European Patent Office
Office européen des brevets

(11) Veröffentlichungsnummer: **0 124 725**
**A1**

(12) # EUROPÄISCHE PATENTANMELDUNG

(21) Anmeldenummer: 84102759.2

(22) Anmeldetag: 14.03.84

(51) Int. Cl.³: C 07 D 309/32
C 07 D 307/58

(30) Priorität: 10.05.83 DE 3317013

(43) Veröffentlichungstag der Anmeldung: 14.11.84 Patentblatt 84/46

(84) Benannte Vertragsstaaten: DE FR GB IT

(71) Anmelder: CHEMISCHE WERKE HÜLS AG
- RSP Patente / PB 15 - Postfach 13 20
D-4370 Marl 1(DE)

(72) Erfinder: Keim, Wilhelm, Prof. Dr.
Brüsseler Ring 99
D-5100 Aachen(DE)

(72) Erfinder: Behr, Arno, Dr.
Heidkopf 7
B-4728 Hergenrath(BE)

(72) Erfinder: Juszak, Klaus-Dieter, Dipl.-Chem.
Martinstrasse 15
D-5100 Aachen(DE)

(54) **Verfahren zur Herstellung von Lactonen.**

(57) Gegenstand der Erfindung sind

1. Verfahren A zur selektiven Herstellung des 2-Ethyliden-6-hepten-5-olids (*1*) durch Umsetzung von Butadien mit $CO_2$,
2. Verfahren B zur Herstellung des 2-Ethyl-2,4-heptadien-4-olids (*2*) aus *1* und
3. Verfahren C zur unmittelbaren Herstellung von *2* aus Butadien und $CO_2$.

Als Katalysatoren werden eingesetzt:

I. $Pd^{2+}$ -Komplexe von β-Dicarbonylverbindungen,
II. kationische Pd-Komplexe mit Allyl- und/oder Dienliganden und $PF_6^-$, $BF_4^-$ oder $ClO_4^-$ als Gegenion,
III. Pd-Komplexe mit Dibenzylidenaceton und
IV. Palladiumbiscarboxylate.

EP 0 124 725 A1

Croydon Printing Company Ltd

## Verfahren zur Herstellung von Lactonen

Die vorliegende Erfindung betrifft Verfahren zur selektiven Herstellung von 2-Ethyliden-6-hepten-5-olid 1 und 2-Ethyl-2,4-heptadien-4-olid 2 in Gegenwart einer Palladiumverbindung und eines tertiären Phosphans.

Lactone und die hieraus herstellbaren Folgeprodukte sind wertvolle organische Produkte mit wichtigen industriellen Anwendungsmöglichkeiten. Von besonderer Bedeutung ist die Verwendung als Aroma- und Geschmackstoff in Lebensmitteln und als Riechstoff in kosmetischen und pharmazeutischen Produkten. So lassen sich z. B. die Lactone 1 und 2 durch Additions-, Substitutions- und andere Folgereaktionen in zahlreiche Derivate überführen, die als Naturstoffe in tierischen Fetten, in Früchten, Getränken sowie in Tabak und Pilzen vorkommen (vgl. Fortschritte d. Chemie org. Naturstoffe 35, 431 - 527, 1978).

Nach Inoue et al. (J. C. S. Chem. Comm. 1976, 605 und Bull. Chem. Soc. Jap. 51, 2375 (1978) erhält man das Lacton 1 in Ausbeuten von 0,4 bis 12,3 % neben mehr als 60 % Butadienoligomeren, wenn man Butadien und Kohlendioxid in polaren aprotischen Lösemitteln in Gegenwart von $Pd(diphos)_2$ umsetzt. Der Tabelle 1 der japanischen Zeitschrift ist zu entnehmen, daß die Ausbeute des Lactons 1 mit 1-Methyl-2-pyrrolidon am besten ist, während sie bei Einsatz von Benzonitril unter 2 % liegt.

Musco fand, daß man bei der Cooligomerisierung von Butadien mit Kohlendioxid in unpolaren Lösemitteln in Gegenwart von Palladiumkomplexen einfach gebundener tertiärer Phosphinliganden ein Gemisch aus dem $\delta$-Lacton 1, den Octadienylestern der (E)-2-Ethylidenhepta-4,6-diensäure und 2-Vinylhepta-4,6-diensäure, 1,3,7-Octatrien und

anderen Produkten erhält (J. C. S. Perkin I 1980, 693 ff.). Es hängt demnach von der Art des Liganden ab, ob und in welchen Ausbeuten das Lacton 1 gebildet wird. Die besten Ergebnisse werden erzielt, wenn man Tricyclohexylphosphan bzw. Triisopropylphosphan verwendet, während gleichzeitig erhebliche Mengen an Estern und Octatrienen gebildet werden. Setzt man (E)-2-Ethylidenhepta-4,6-diensäure in Gegenwart von Palladium-tert.-phosphin-komplexen um, erhält man das γ-Lacton 2 sowie dessen 2-Ethyliden-Isomeres. Die besten Ausbeuten an 2 bei dieser Isomerisierung betragen 42 %.

Gegenstand der DE-OS 28 38 610 ist ein Verfahren zur Herstellung von Octadienylestern isomerer Nonatriensäuren und des δ-Lactons 1 aus Butadien und Kohlendioxid in Gegenwart eines Palladiumphosphorkomplexes der Formel $Pd(PR_3)_x$ mit x = 2, 3 und 4, wobei R ein Alkyl-, Cycloalkyl- oder Phenylrest sein kann. Soll das Lacton 1 Hauptprodukt werden, arbeitet man vorzugsweise bei einem Druck zwischen 250 und 300 bar und bei einer Temperatur zwischen 60 und 85 °C. Doch selbst unter diesen Bedingungen läßt sich die Bildung beträchtlicher Mengen an Butadienoligomeren nicht vermeiden.

Schließlich beschreibt die EP-OS 0 050 445 ein Verfahren zur Herstellung des δ-Lactons 1 aus Butadien und Kohlendioxid, wobei als Katalysator eine komplexe Palladiumverbindung, ein tertiäres Amin und gegebenenfalls zusätzlich ein weiteres Trialkylphosphan und/oder Hydrochinon eingesetzt wird. Die Palladiumverbindung ist nur durch eine aufwendige Synthese aus einem Palladiumsalz, einer Verbindung des 3-wertigen Phosphors oder Pyridin oder einem Pyridinderivat und gegebenenfalls einer einfach oder mehrfach ungesättigten Verbindung, wie beispielsweise Chinon, herstellbar. Man führt die Reaktion in einem polaren aprotischen Lösemittel, wie Acetonitril, vorzugsweise bei einem Durck von 10 bis 50 bar und einer Temperatur von 40 bis 80 °C durch. Dieses Verfahren eröffnet erstmalig einen präparativ gangbaren Weg zur Herstellung von 1.

Ein großer Nachteil aller Verfahren nach dem Stand der Technik besteht darin, daß sie keine für den technischen Maßstab geeignete Aufarbeitungsmethode aufzeigen. Es wird lediglich beschrieben, daß das erhaltene Reaktionsgemisch im analytischen Maßstab durch Hochdruckflüssigkeitschromatographie oder Säulenchromatographie aufgetrennt werden kann.

Ein Ziel der vorliegenden Erfindung war es, ein gewerblich nutzbares Verfahren A zur Herstellung des Lactons 1 aus Butadien und Kohlendioxid zu entwickeln. Weitere Ziele waren die Entwicklung einfach herzustellender Katalysatoren sowie die Auffindung technischer Methoden zur Isolierung von 1. Dies gelang mit Hilfe der in den Ansprüchen 1 und 4 bis 8 aufgeführten Verfahren.

Gegenstand der Erfindung ist auch ein Verfahren B zur Herstellung des Lactons 2 aus dem Lacton 1, wobei das molare Verhältnis des eingesetzten Lactons 1 zu dem in den Katalysatoren enthaltenem Palladium zwischen 10 und 100 zu 1 liegt.

Schließlich betrifft die Erfindung auch ein Verfahren C zur Herstellung des Lactons 2 aus Butadien und Kohlendioxid mit Hilfe der gleichen Katalysatoren.

Die Verfahren A, B und C ergeben mit überraschend hoher Selektivität die Lactone 1 bzw. 2. So entstehen nach dem Verfahren A weniger als 10 % Nebenprodukte wie Butadienoligomere und Octadienylester. Die Selektivität des Verfahrens B liegt bei über 97 %. Das Verfahren C eröffnet erstmalig einen unmittelbaren Zugang des Lactons 2 aus Butadien und Kohlendioxid. Überraschend erscheint der Befund, daß es lediglich von der Menge des eingesetzten Katalysators abhängt, ob bei Reaktion von Butadien und Kohlendioxid das Lacton 1 oder 2 gebildet wird. Ein weiterer Vorteil der vorstehenden Verfahren ist

die bequeme destillative Abtrennung der Lactone 1 bzw. 2 aus den entsprechenden Reaktionsgemischen.

Der Katalysator der erfindungsgemäßen Verfahren A, B und C besteht aus einer der im folgenden näher beschriebenen Palladiumverbindungen und einem Liganden.

I. Komplexe des zweiwertigen Palladiums mit 2 Molen einer ß-Dicarbonylverbindung der Formel

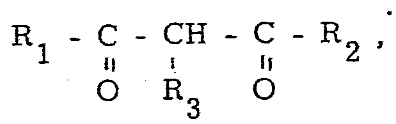

wobei $R_1$ und $R_2$ gleiche oder verschiedene $(C_1-C_3)$-Alkylreste oder den Phenylrest bedeuten können und $R_3$ ein $(C_1-C_3)$-Alkylrest oder vorzugsweise Wasserstoff sein kann. Besonders geeignet ist Acetylaceton. Derartige Komplexe sind nach der DE-PS 24 02 383 erhältlich.

II. Kationische Palladiumkomplexe, die als Liganden das Allyl- und/oder Diensystem enthalten. Typische Verbindungsklassen umfassen dienhaltige Palladiumverbindungen, die als zusätzlichen Liganden eine ß-Dicarbonylverbindung, einen offenkettigen oder cyclischen Enylrest oder das Cyclopentadienylsystem (cp) enthalten. Vorzugsweise kommt als Dienligand das Cyclooctadien-1,5 (COD) in Betracht. Als Gegenion werden hauptsächlich der Hexafluorphosphat-, der Tetrafluorborat- und der Perchloratrest eingesetzt. Beispielhaft seien aufgeführt:

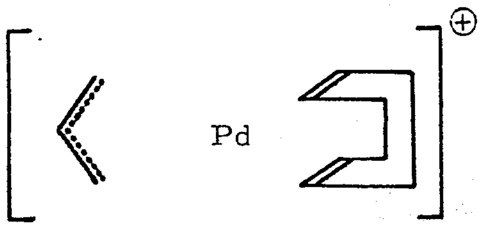

$BF_4^{\ominus}$

(Inorg. Syntheses 13, 61 (1972)

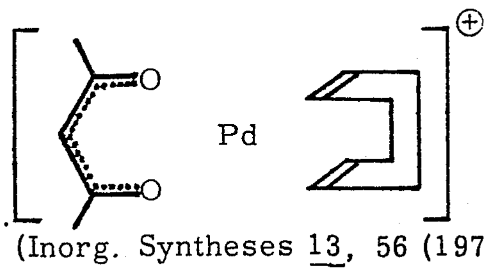

$BF_4^{\ominus}$

(Inorg. Syntheses 13, 56 (1972)

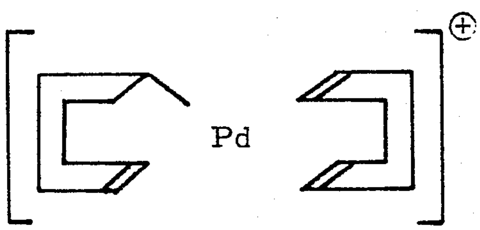

$BF_4^{\ominus}$

(J. Chem. Soc., Dalton Trans. 1977, 2228)

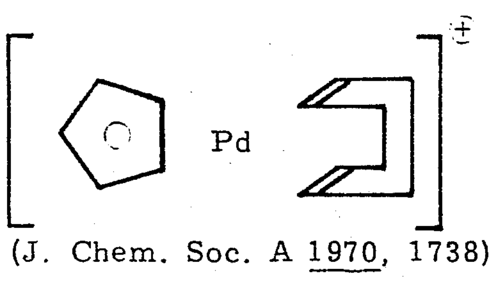

$BF_4^{\ominus}$

(J. Chem. Soc. A 1970, 1738)

III. Palladium(O)-komplex aus Palladium und 2 Molen Dibenzylidenaceton (dba). Die Struktur eines derartigen Komplexes ist nicht gesichert. Die Synthese wird in Inorg. Syntheses 17, 135 (1977) beschrieben.

IV. Palladiumbiscarboxylate der Struktur

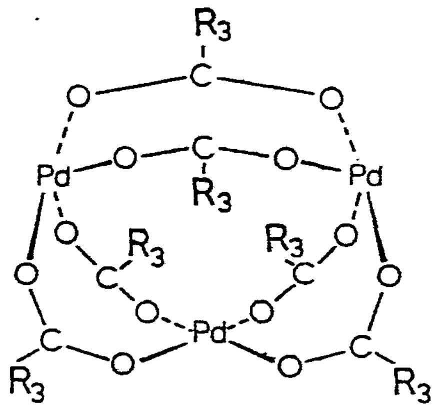

Vorzugsweise hat $R_3$ die Bedeutung von Methyl. Die Synthese wird in J. Chem. Soc. 1965, 3632, beschrieben.

Der Ligand ist ein Trialkylphosphan der Formel $P(R_6)_3$, wobei $R_6$ einen in $\alpha$-Stellung verzweigten (Cyclo)Alkylrest darstellt. In erster Linie kommen $C_3$-$C_{12}$-Alkylgruppen oder $C_5$-$C_8$-Cycloalkylgruppen infrage. Bevorzugte Phosphane sind das Tri-(cyclohexyl)-phosphan $PCy_3$ und insbesondere das Tri-(isopropyl)-phosphan $PPr^i_3$.

Das molare Verhältnis von Palladium zum Phosphor liegt zwischen 1 : 1 und 1 : 10, vorzugsweise zwischen 1 : 1,5 und 1 : 3,5.

Das erfindungsgemäße Verfahren A (Herstellung von 1) wird in polaren aprotischen Lösemitteln in homogener flüssiger Phase durchgeführt. Geeignet sind Nitrile, insbesondere die technisch leicht zugänglichen Mono- und Dinitrile mit bis zu 8 C-Atomen. Bevorzugt werden Benzonitril, Propionitril und insbesondere Acetonitril.

Pro l Lösemittel werden 100 bis 1 000 g, vorzugsweise 400 bis 500 g, Butadien eingesetzt.

Das vergleichsweise billige Kohlendioxid wird mindestens in der stöchiometrischen Menge, aus praktischen Erwägungen jedoch vorteilhafterweise in geringem Überschuß, eingesetzt.

Das molare Verhältnis von Butadien zu Palladium kann im Bereich von 500 : 1 bis 15 000 : 1 liegen, bevorzugt wird ein Verhältnis von 1 000 : 1 bis 2 000 : 1 eingehalten.

Das Verfahren A wird bei einer Temperatur zwischen 50 und 140 °C, vorzugsweise 70 bis 100 °C, durchgeführt. Am günstigsten hat sich eine Temperatur zwischen 80 und 95 °C erwiesen. Die Reaktion wird unter erhöhtem Druck durchgeführt. Der bevorzugte Druckbereich liegt zwischen 40 und 60 bar. Die Reaktionszeit beträgt mindestens 2, vorzugsweise 10 bis 20 Stunden.

Eine praktische Ausführung des Verfahrens A wird im folgenden beschrieben:

In ein Glasgefäß wird unter Schutzgasatmosphäre die Palladiumverbindung sowie das tertiäre Phosphan eingewogen. Das Lösemittel wird zugegeben und so lange gerührt, bis sich eine homogene flüssige Phase bildet. Diese Katalysatorlösung wird in einen mit Argon oder Stickstoff gefüllten Autoklaven überführt. Die benötigte Menge Butadien wird nach Trocknung über 4 Å-Molsieb in einem Gefäß auskondensiert und dann in den Autoklaven überführt. Nachfolgend wird das Kohlendioxid aufgepreßt und der Autoklav auf die gewünschte Temperatur gebracht. Nach Beendigung der Reaktion und Abkühlung auf Raumtemperatur wird der Autoklav entspannt, die Reaktionslösung entnommen und das Lösemittel im Wasserstrahlvakuum abdestilliert. Die verbleibende Reaktionslösung wird unter Zusatz eines Standards gaschromatographisch analysiert. Die Destillation des Reaktionsgemisches liefert das 2-Ethyliden-6-hepten-5-olid in einer Reinheit von über 98 % (Siedepunkt: 76 °C bei $10^{-2}$ mm Hg; $n_D^{20}$ = 1,5013). Bei der Destillation ist darauf zu achten, daß die Blasentemperatur nicht über 100 °C steigt, da sonst Isomerisierungen ablaufen. Man kann die abschließende Destillation auch in Form einer

Wasserdampfdestillation bei Normaldruck durchführen. So ergibt beispielsweise das Reaktionsgemisch nach Durchleiten von Wasser in Form von Wasserdampf und Extraktion der Wasserphase mit Diethylether 1 in einer Reinheit von 97 %.

Das erfindungsgemäße Verfahren B zur Darstellung des Lactons 2 durch Isomerisierung von 1 wird in Gegenwart des bereits beschriebenen Gemisches aus den Palladiumverbindungen und dem Liganden durchgeführt.

Das molare Verhältnis des eingesetzten Lactons 1 zum Palladium liegt zwischen 10 : 1 und 100 : 1, vorzugsweise zwischen 15 : 1 und 25 : 1.

Es kommen die gleichen Lösemittel zum Einsatz, die bereits im Verfahren A genannt wurden. Üblicherweise werden pro l Lösemittel 5 bis 100 g Lacton 1 eingesetzt, vorzugsweise sind es 20 bis 50 g.

Das Verfahren B wird bei einer Temperatur zwischen 70 und 100 °C, vorzugsweise zwischen 85 und 95 °C, durchgeführt. Die Reaktion kann bei Normaldruck oder Überdruck, vorzugsweise bei dem sich bei der jeweiligen Temperatur einstellenden Eigendruck von 1,5 bis 3,5 bar durchgeführt werden. Die Reaktionszeit beträgt mindestens 2, vorzugsweise 10 bis 20 Stunden. Längere Reaktionszeiten führen nicht zu einer Zersetzung des Produktes. Im folgenden wird eine praktische Ausführungsform des Verfahrens beschrieben:

In ein Glasgefäß wird die Palladiumverbindung sowie das tertiäre Phosphan eingewogen. Das Lösemittel wird zugegeben und so lange gerührt, bis sich eine homogene flüssige Phase bildet. Zu dieser Katalysatorlösung wird die Verbindung 1 gegeben. Nach der Reaktion wird das Lösemittel abdestilliert und die Verbindung 2 in einer Reinheit von 97 % (Siedepunkt: 80 °C bei $10^{-2}$ mm Hg) fraktioniert.

Auch das erfindungsgemäße Verfahren C wird mit Hilfe des Gemisches aus Palladiumverbindungen und Liganden durchgeführt. Das molare Verhältnis des eingesetzten Butadiens zum Palladium liegt zwischen 800 : 1 und 10 : 1, vorzugsweise zwischen 200 : 1 und 50 : 1.

Als Lösemittel kommen in erster Linie wiederum die Nitrile in Betracht, die bereits beim Verfahren A genannt wurden. Pro l Lösemittel werden 100 bis 1 000 g, vorzugsweise 400 bis 500 g, Butadien eingesetzt. Die Temperatur liegt zwischen 70 und 120 °C, zweckmäßigerweise zwischen 85 und 95 °C. Der Druck entspricht dem des Verfahrens A. Die Reaktionszeit beträgt mindestens 2, vorzugsweise 10 bis 40 Stunden. Das bei der Reaktion nicht umgesetzte Butadien kann problemlos abgetrennt und für einen neuen Ansatz verwendet werden.

Beispiel 1 (Verfahren A)

14 g (0,26 Mol) Butadien, 15 g (0,34 Mol) Kohlendioxid, 0,05 g (0,16 mMol) Palladiumbis(acetylacetonat) $Pd(acac)_2$, 0,08 g (0,48 mMol Tri-(isopropyl)-phosphan $PPr^i_3$ und 30 ml Acetonitril werden in einen 75 ml-V4A-Stahlautoklaven überführt. Beim Erhitzen auf 90 °C steigt der Druck um 25 bar auf 50 bar an. Nach der Reaktionszeit von 15 Stunden wird der Autoklav auf Raumtemperatur abgekühlt und das nicht reagierte Kohlendioxid und Butadien sowie das Lösungsmittel bei einem Vakuum von 15 mm Hg entfernt. Die gaschromatographische Analyse des Rückstands ergibt eine Ausbeute an 2-Ethyliden-6-hepten-5-olid (1) von 39,5 Mol-%, bezogen auf eingesetztes Butadien. Isomere Lactone werden in einer Ausbeute unter 0,5 Mol-% gebildet, die Octadienylester der 2-Ethylidenhepta-4,6-diensäure zu 1,3 Mol-%. Durch Destillation des Rückstands können 7,54 g (50 mMol) 2-Ethyliden-6-hepten-5-olid (1) in einer Reinheit von 98 % erhalten werden.

Die Identifizierung des 2-Ethyliden-6-hepten-5-olids erfolgt durch Protonen-NMR-, Infrarot- und Massenspektrometrie:

$^1$H-NMR (90 MHz, $CDCl_3$): $\delta$ = 7,05 (m, 1H, $C=CH-CH_3$), 5,78 (m, 1H, $CH_2-CH-CH$), 5,3 (d, 1H, $H_2C=CH$, trans), 5,15 (d, 1H, $H_2C-CH$, cis), 4,75 (m, 1H, CH-CH-O), 2,50 (m, 2H, $CH_2-CH_2-C=C$), 2,00 (m, 2H, $CH-CH_2-CH_2$), 1,75 (d, 3H, $CH-CH_3$).
IR (Film): $\nu$ = 3005, 2940, 2920, 2860, 1760, 1680, 1140, 1380, 1210, 1020, 980, 720 $cm^{-1}$.
MS: m/e = 152 ($M^+$, 10 %), 137 (11 %), 124 (30 %), 104 (22 %), 96 (100 %), 81 (41 %), 68 (87 %), 67 (70 %), 55 (31 %), 54 (62 %), 53 (41 %), 41 (47 %), 39 (78 %).

Beispiele 2 bis 8 (Verfahren A)

Gemäß Beispiel 1 wird in den Beispielen 2 bis 8 verfahren. Als Katalysator wird das System $Pd(acac)_2/PPr^i_3$ im Lösungsmittel Acetonitril eingesetzt. Die Ergebnisse sind in Tabelle 1 zusammengefaßt.

Tabelle 1

260 mMol Butadien; 340 mMol Kohlendioxid; 30 ml Acetonitril; T = 90 °C

| Bsp. | $Pd(acac)_2$ mMol | $PPr^i_3$ mMol | t h | Ausbeute an 1 (%) | Selektivität+) |
|---|---|---|---|---|---|
| 2 | 0,32 | 0,96 | 15 | 35,0 | 89,8 |
| 3 | 0,08 | 0,24 | 15 | 31,5 | 96,6 |
| 4 | 0,16 | 0,32 | 15 | 36,5 | 97,1 |
| 5 | 0,16 | 0,40 | 15 | 39,1 | 96,2 |
| 6 | 0,16 | 0,56 | 15 | 39,3 | 93,0 |
| 7 | 0,16 | 0,48 | 11 | 37,8 | 96,6 |
| 8 | 0,16 | 0,48 | 65 | 47,2 | 92,1 |

+) bezogen auf alle Butadien-$CO_2$-Folgeprodukte in Gewichtsprozent

Beispiele 9 bis 11 und A bis C (Verfahren A)

Gemäß Beispiel 1 wird in den Beispielen 9 bis 11 und A bis C verfahren. Beispiel A zeigt, daß bei Verwendung von Trialkylphosphanen, deren Alkylgruppen nicht in α-Stellung verzweigt sind, Selektivität und Ausbeute drastisch absinken. Die Beispiele B und C zeigen, daß Lösemittel ohne Nitrilgruppe zu niedrigeren Ausbeuten führen. Die Ergebnisse gehen im einzelnen aus Tabelle 2 hervor.

Tabelle 2

260 mMol Butadien; 340 mMol Kohlendioxid; 0,16 mMol $Pd(acac)_2$; 0,48 mMol Ligand; 30 ml Lösungsmittel; T = 90 °C; t = 15 h

| Bsp. | Ligand | Lösungsmittel | Ausbeute an 1 (%) | Selektivität+) |
|---|---|---|---|---|
| 9 | $PCy_3$ | Acetonitril | 38,6 | 95,9 |
| 10 | $PPr^i_3$ | Propionitril | 35,6 | 95,9 |
| 11 | $PPr^i_3$ | Benzonitril | 16,4 | 98,8 |
| A | $PEt_3$ | Acetonitril | 3,9 | 22,4 |
| B | $PPr^i_3$ | Aceton | 1,9 | 84,2 |
| C | $PPr^i_3$ | Tetrahydrofuran | 0,2 | 66,7 |

+) bezogen auf alle Butadien-$CO_2$-Folgeprodukte in Gewichtsprozent

Beispiele 12 bis 14 (Verfahren A)

Gemäß Beispiel 1 wird in den Beispielen 12 bis 14 verfahren. Es wurden jeweils 260 mMol Butadien und 340 mMol Kohlendioxid eingesetzt. Die Temperatur betrug 90 °C. Die Reaktionszeit betrug 15 Stunden.

Tabelle 3

| Bsp. | Komplex | Ligand | Ausbeute an 1 (%) | Selektivität+) |
|---|---|---|---|---|
| 12*) | $Pd(dba)_2$ | $PCy_3$ | 35 | 96,6 |
| 13*) | $[Pd(COD)(cp)]^{\oplus} BF_4^{\ominus}$ | $PPr^i_3$ | 27 | 91,7 |
| 14+) | $[Pd_3(OAc)_6]$ | $PPr^i_3$ | 11 | 60,3 |

+) bezogen auf alle Butadien-$CO_2$-Folgeprodukte in Gewichtsprozent

*) Es wurden 0,65 mMol Palladiumverbindung und 1,95 mMol Ligand eingesetzt.

Beispiel 15 (Verfahren B)

10 g (0,066 Mol) der Verbindung 1, 1 g (0,0032 Mol) $Pd(acac)_2$, 1,6 g (0,0098 Mol) $PPr^i_3$ und 200 ml Acetonitril werden in einem Glasautoklaven 15 Stunden lang bei 90 °C gerührt. Nach der Reaktion wird abgekühlt und das Lösemittel bei einem Vakuum von 15 mm Hg entfernt. Die gaschromatographische Analyse des Rückstands ergibt eine Ausbeute an 2 von 90 %. Durch Destillation wird die Verbindung 2 in einer Reinheit von 97 % erhalten.

Die Identifizierung des 2-Ethyl-2,4-heptadien-4-olids (2) erfolgt u. a. durch Protonen-NMR-, Infrarot- und Massenspektroskopie:

$^1H$-NMR (90 MHz, $CDCl_3$): $\delta$ = 6,88 (s, 1H, C=CH-C=), 5,08 (t, 1H, $CH_2$-HC=C), 2,38 (m, 4H, $-CH_2-$), 1,18 (t, 3H, $CH_3-CH_2$-CH=), 1,05 (t, 3H, $CH_3-CH_2$-C=).
IR (Film): $\nu$ = 3095, 2980, 2940, 2880, 1770, 1675, 1615, 1460, 1230, 1040, 780 $cm^{-1}$.

MS: m/e = 152 ($M^+$, 40 %), 137 (28 %), 124 (10 %), 110 (100 %), 109 (14 %), 96 (12 %), 82 (94 %), 69 (16 %), 55 (85 %), 54 (63 %), 53 (21 %), 41 (30 %), 39 (68 %).

Beispiel 16 (Verfahren C)

Analog zu Beispiel 1 werden 14,8 g (0,27 Mol) Butadien, 13,8 g (0,30 Mol) Kohlendioxid, 0,95 g (3,2 mMol) $Pd(acac)_2$ und 1,5 g (9,38 mMol) $PPr^i_3$ sowie 30 ml Acetonitril in einen 75 ml-V4A-Stahlautoklaven überführt, auf 90 °C erhitzt und 15 Stunden bei dieser Temperatur gehalten. Anschließend wird der Autoklav auf Raumtemperatur abgekühlt und der Inhalt analog zu Beispiel 1 aufgearbeitet. Man erhält das Lacton 2 in einer Ausbeute von 17 %. Die Selektivität innerhalb der Butadien-$CO_2$-Additionsprodukte beträgt 97 %. Die spektroskopischen Daten entsprechen der Probe, die nach Beispiel 15 erhalten wird.

Patentansprüche:

1. Verfahren A zur selektiven Herstellung des 2-Ethyliden-6-hepten-5-olis (1) durch Umsetzung von Butadien mit Kohlendioxid in Gegenwart eines Katalysators, der einen Palladiumkomplex und eine P(III)-Verbindung als Liganden enthält, in einem polaren aprotischen Lösemittel im Temperaturbereich von 20 bis 100 °C und bei einem Druck über 10 bar,
dadurch gekennzeichnet,
daß der Palladiumkomplex eine der im folgenden aufgeführten Gruppen umfaßt:

I. $Pd^{2+}$-Komplexe einer ß-Dicarbonylverbindung der Formel

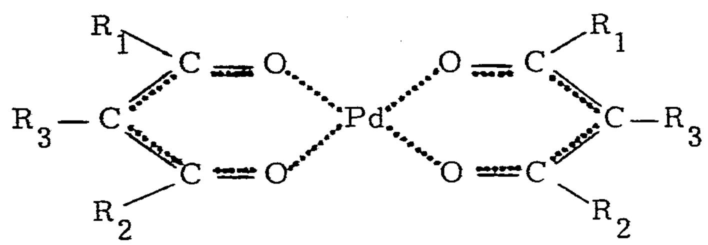,

wobei $R_1$ und $R_2$ gleiche oder verschiedene $(C_1-C_3)$-Alkylreste oder den Phenylrest darstellen können und $R_3$ ein $(C_1-C_3)$-Alkylrest oder Wasserstoff sein kann,

II. kationische Palladiumkomplexe, die

1. $PF_6^{\ominus}$, $BF_4^{\ominus}$ oder $ClO_4^{\ominus}$ als Gegenion enthalten, die

2. Allyl- und/oder Dienliganden enthalten,
- wobei das Allylsystem die Struktur

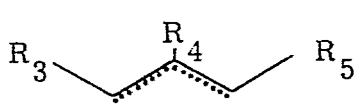

aufweist und $R_4$ und $R_5$ die Bedeutung von Wasserstoff oder eines Alkylrestes mit bis zu 3 C-Atomen haben und

- wobei als Diene offenkettige oder cyclische Verbindungen mit bis zu 12 C-Atomen geeignet sind und die

3. als weitere Liganden eine $C_4$-$C_8$-ß-Dicarbonylverbindung, einen $C_3$-$C_{12}$-Enylrest oder das Cyclopentadienylsystem enthalten,

III. Palladiumkomplexe aus Palladium und 2Molen Dibenzylidenaceton und

IV. Palladiumbiscarboxylate der Formel $[Pd_3(OOCR_3)_6]$,

daß der Ligand ein Trialkylphosphan der Formel $P(R_6)_3$ ist und $R_6$ einen in $\alpha$-Stellung verzweigten Alkylrest mit 3 bis 12 C-Atomen oder einen Cycloalkylrest mit 5 bis 8 C-Atomen darstellt und daß das molare Verhältnis von Butadien zu Palladium zwischen 1 000 und 2 000 : 1 liegt.

2. Verfahren B zur Herstellung des 2-Ethyl-2,4-heptadien-4-olids (2), dadurch gekennzeichnet,
daß man 2-Ethyliden-6-hepten-5-olid (1) mit den in Anspruch 1 genannten Katalysatoren umsetzt, wobei das molare Verhältnis von 1 zum Palladium zwischen 10 : 1 und 100 : 1, vorzugsweise zwischen 15 : 1 und 25 : 1, liegt.

3. Verfahren C zur Herstellung des 2-Ethyl-2,4-heptadien-4-olids (2), dadurch gekennzeichnet,
daß man ebenso wie in Anspruch 1 Butadien und Kohlendioxid in Gegenwart eines Katalysators aus einer Palladiumverbindung und einem Liganden umsetzt und wobei das molare Verhältnis von Butadien zu Palladium zwischen 800 : 1 und 10 : 1, vorzugsweise zwischen 200 : 1 und 50 : 1, liegt.

4. Verfahren nach den Ansprüchen 1, 2 oder 3, dadurch gekennzeichnet, daß bei den $Pd^{2+}$-Komplexen gemäß I $R_1$ und $R_2$ die Bedeutung von $CH_3$ haben und $R_3$ Wasserstoff ist.

5. Verfahren nach Anspruch 4, dadurch gekennzeichnet, daß das molare Verhältnis zwischen Palladium und Phosphor zwischen 1 : 2 und 1 : 4 liegt.

6. Verfahren nach den Ansprüchen 1, 2 oder 3, dadurch gekennzeichnet, daß der Ligand das Tri-(cyclohexyl)-phosphan oder vorzugsweise das Tri-(isopropyl)-phosphan ist.

7. Verfahren nach den Ansprüchen 1 bis 6, dadurch gekennzeichnet, daß man das bei der Reaktion anfallende Reaktionsgemisch im Vakuum bei einer Temperatur unterhalb von 100 °C destilliert.

8. Verfahren nach den Ansprüchen 1 bis 6, dadurch gekennzeichnet, daß man das erhaltene Reaktionsgemisch einer Wasserdampfdestillation unterwirft.

Europäisches Patentamt

# EUROPÄISCHER RECHERCHENBERICHT

EP 84102759.2

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl. 3) |
|---|---|---|---|
| A | CHEMICAL ABSTRACTS, Vol. 89, No. 15, 9. Oktober 1978, Columbus, Ohio, USA<br>MUSCO, A.; PEREGO, C.; TARTIARI, V. "Telomerization reactions of butadiene and carbon dioxide catalyzed by phosphine palladium (0) complexes: (E)-2-ethylidene-hept-6-en-5-olide and octadienyl esters of 2-ethylidenehepta-4,6-dienoic acid."<br>Seite 545, Spalte 1, Zusammenfassung No. 128 999n<br>& Inorg. Chim. Acta 1978, 28(2), L147-L148 (Eng.)<br>-- | 1 | C 07 D 309/32<br>C 07 D 307/58 |
| A | DE - A1 - 2 827 383 (FIRMENICH)<br>* Formel I *<br>---- | 2,3 | |
| | | | **RECHERCHIERTE SACHGEBIETE (Int. Cl. 3)** |
| | | | C 07 D 309/00<br>C 07 D 307/00 |

Der vorliegende Recherchenbericht wurde für alle Patentansprüche erstellt.

| Recherchenort | Abschlußdatum der Recherche | Prüfer |
|---|---|---|
| WIEN | 24-07-1984 | HAMMER |

KATEGORIE DER GENANNTEN DOKUMENTEN

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument